# EUROPEAN PATENT SPECIFICATION

(11) **EP 1 374 899 B1**
(45) Date of publication and mention of the grant of the patent: **27.09.2006**
(21) Application number: 02705269.5
(22) Date of filing: 15.03.2002
(51) Int. Cl.: A61K 31/5575, A61P 25/00

(54) **REMEDIES FOR POST-TRAUMATIC STRESS DISORDER**
MITTEL GEGEN POSTTRAUMATISCHE STRESSZUSTÄNDE
REMEDES POUR TROUBLE DE STRESS POST-TRAUMATIQUE

(30) Priority: 16.03.2001 JP 2001075398
(43) Date of publication of application: 02.01.2004
(73) Proprietor: Kyoto University, Kyoto-shi, Kyoto 606-8501 (JP)
(72) Inventor: NARUMIYA, Shuh, Kyoto-shi, Kyoto 606-0007 (JP); MARUYAMA, Takayuki, Ono Pharmaceutical Co., Ltd., Mishima-gun, Osaka 618-8585 (JP)
(74) Representative: Henkel, Feiler & Hänzel
(86) International application number: PCT/JP2002/002513
(87) International publication number: WO 2002/076505

(56) References cited:
- JP-A- 11 322 709
- TREPTOW K. ET AL.: 'Effect of prostaglandins (PGE1 and PGE2) on central-nervous processes of conditional reflectory learning and the formation of a time-memory' ACTA BIOL. MED. GER. vol. 31, no. 1, 1973, pages 111 - 120, XP002954419
- RANDALL P.K. ET AL.: 'Effect of the benzodiazepine antagonist flumazenil in PTSD' BIOL. PSYCHIATRY vol. 38, no. 5, 1995, pages 319 - 324, XP002954420

## Description

### TECHNICAL FIELD

This invention relates medicines and prophylactic drugs of post-traumatic stress disorder which contain EP₁ agonist as an active ingredient.

### BACKGROUND OF THE INVENTION

Post-Traumatic Stress Disorder is called PTSD(Post-Traumatic Stress Disorder), and located to anxiety neurosis caused by receiving psychological bruises by experience beyond a usual corrective ability against experience of wars, natural disasters, domestic violences or sexual abuses, etc. By imaging diagnosis of center nerve, it is understood that not only psychological sides, shrinkage of hippocampus and dysfunction of prefrontal cortex has happened and the number of benzodiazepine receptors has decreased.

And, it is reported that accommodation disorder of neurotransmitter in brain has happened in a physiologic research (Semin Clin Neuropsychiatry, 4(4), 242-248 (1999)).

On the other hand, prostaglandin E₂ (hereafter, it is abbreviated to PGE₂) is known as metabolic products in arachidonic acid cascade and to have cytoprotective effects, oxytocic effects, algogenic effects, peristalsis movement promotion of digestive tract, arousal effects, suppressive effects of gastrin releasing , hypotensive effects, and diuresis,etc.

By the recent researches, it has been understood that subtypes with each different role exist in PGE₂ receptors. Subtypes known now have been divided roughly and called EP₁, EP₂, EP₃, and EP₄, respectively(Negishi M. et al, J. Lipid Mediators Cell Signaling, 12, 379-391(1995)).

Though PGE₂ possess the fault that actions other than aimed action become side effects because of thier many bioactivations, researches, which overcome this fault by the examination of the role of each subtype and obtaining effective compounds to the subtype only, is continued.

It is known that EP₁ receptor among these subtypes takes part in having pain, heating, and diuretic(Br. J. Pharmacol. 1994, 112, 735-40, and European J. Pharmacol., 152(1988) 273-279, Gen Pharmacol., Sep 1992, 23(5), p805-809, reference.). Therefore, it is thought that antagonists to this receptor are effective as analgesics, antipyretic drugs, and medicines of pollakisuria.

Recently, EP₁ receptor-deficient mice have been made, and the various examinations have been done. For example, it has been known that formations of intraintestinal polypus and abnormal crypt of large intestine mucous membrane induced by a chemical carcinogen (azoxymethane) in EP₁ receptor-deficient mice partially decrease(reference to WO 00/69465).

### DISCLOSURE OF THE INVENTION

These inventors found that EP₁ receptor-deficient mice have common features to a diagnosis standard of PTSD(Pitman, R.K.; Overviews of biological themes in PTSD. Ann. N. Y. Acad. Sci. 821, 1-9, 1997), as a result of their various experiments by use of EP₁ receptor-deficient mice and their zealous researches to investigate functions of EP₁ receptor. Moreover, because of obtaining a result similar to that of EP₁ receptor-deficient mice when EP₁ antagonists are administered to normal mice, it has been understood that EP₁ receptor takes part in PTSD.

Therefore, it became clear that the EP₁ agonists could be useful for treatment and prevention of PTSD, and these inventors completed this invention.

The present invention, thus, provides a use of a EP₁ agonist in the manufacture of a medicament for the treatment of post-traumatic stress disorder (PTSD).

A preferred embodiment thereof is claimed in claim 2.

Though facts obtained from the experiments was easily summarized as follows, EP₁ receptor-deficient mice presented the following symptoms compared to normal mice.
1) ACTH(adrenocorticotrophic hormone) production caused by lipopolysaccharide(LPS) had significantly decreased.
2) The level of decrease in spontaneous locomotor activity caused by LPS was a little.
3) The acoustic startle reaction had been accelerated.
4) In induce of aggressiveness by electrical shock, aggressiveness had been accelerated.
5) Acts of smelling, licking, tracking, tidying, riding, and diving to other young mice were hardly seen, and they was indifferent.
6) The action of jumping off a high platform was seen.
7) The metabolism of dopamine had been accelerated in lobus frontalis of cerebral cortex and striate corpus.

It was confirmed that normal mice to which EP₁ antagonists had been administered showed symptoms similar to 1), 2), 5), and 6) at least. Therefor, it is obvious that EP₁ agonists that show the action opposite to antagonists could be useful for PTSD.

On the other hand, though the influence of EP₁ receptor on anxiety and memory, etc. was examined, it was confirmed that EP₁ receptor did not influence to them. That is, though the anxiety action was examined by the tight/dark box test (a method of assuming frequency and time that moves from dark place to bright place to be index of anxiety), the open-field test(H.Miyagawa,et al; Behev. Brain Res., 91, 73-81, 1998), and the elevated plus maze test (K. Yamada,et al; J. Neuroimmunol., 111, 131-138, 2000), EP₁ receptor-deficient mice almost showed a similar response to normal mice. Moreover, though the influence on exploratory behavior and the short-term memory had been examined by the Y-maze test(K. Yamada,et al; Eur. J. Pharmacol. 349, 15-22, 1998), the action of EP₁ receptor-deficient mice was similar to that of normal mice. Thus, it was confirmed that EP₁ receptor do not harmfully influence to anxiety and memory.

EP₁ agonists in this invention indicate what to bind to EP₁ receptor and activate it. For example, (13E)-(11α, 15S, 17S)-2, 5-ethano-6, 9-dioxo-11, 15-dehydro-17, 20-dimethylprosta-13-enoic acid(JP11322709), etc., are known as a selective EP₁ agonist, and belongs within the range of this invention.
Moreover, though there is Sulprostone (CAS No.60325-46-4), which is not a selective compound to EP₁ receptor, as selective compounds to both EP₁ and EP3 receptor, and it belongs within the range of this invention.

And, because PGE₁ and PGE₂, etc., have agonistic activities to EP₁ receptor though they are not selective, it is thought that they could be used to treat PTSD.

### [Toxicity]

It has been confirmed that the above compounds could be safe enough to use as medicines.

### INDUSTRIAL APPLICABILITY

### [Application to medicine]

When EP₁ agonists, which are the compounds used according to this invention, or the nontoxicity salt are used by the purpose of treatment and prevention of PTSD, usually, they are systemically or locally administered as oral or non-oral agents.

The dosage is different depending on age, weight, symptom, therapeutic effect, administration mode, and processing time, etc., and they are orally administered once or several times a day within the range of the dosage from 1 mg to 100mg for adult, or are parenterally administered (Intravenous administration, desirably) within the range of the dosage from 0.1 mg to 10mg or are intravenously administered for 1 hour to 24 hours a day continually.

Of course, as the above, because the dosage changes according to various conditions, it might be enough in less amount than the above dosage or might be necessary beyond the range.
When the compounds used according to this invention are administered, they will be used as solid medicines, liquid compositions, and other compositions for the oral administration, injection drugs, external preparations, and suppositories, etc., for the parenteral administration.

The tablet, the pill, the capsule, the powder, and the granule, etc., are included in the solid compositions for the oral administration. The hard capsules and the soft capsule are included in the capsule.

In such solid medicine, one or more activators are mixed with at least an inert diluent, for example, lactose, mannitol, glucose, hydroxypropylcellulose, microcrystalline cellulose, starch, or magnesium aluminometasilicate. The composition may contain additives besides inert diluent, for example, lubricants such as magnesium stearate, disintegrators such as cellulose glycolic acid calcium, solubilizers such as glutamate or aspartic acid according to the usual method. The tablets or the pills, if necessary, may be coated with films that consists of a stomach soluble or intestines soluble materials such as saccharose gelatin, hydroxypropylcellulose, or hydroxypropylmethylcellulose phthalate, etc., or may be coated with layers of two or more. In addition, the capsules of materials absorbed such as gelatin are also included.

The liquid compositions for oral administration contain opalizers, solutions, syrup drugs, and the elixir, etc., allowed as medicines. On such liquid compositions, one or more activators is/are contained into inert diluents(for example, purified water or ethanol) used generally. This composition may contain penetrants, auxiliary materials such as suspension, emulsifying agents, sweeteners, flavor medicines, aromatic substances, or preservatives besides inert diluents.

In other compositions for oral administration, one or more activators and aerosols, which are prescribed by a method well-known itself, are contained. This composition may contain stabilizers such as sodium hydrogen sulfite, stabilizers that give isotonicity, or isotonic agents such as sodium chloride, sodium citrate or citrates besides inert diluents. The manufacturing method of aerosols have been described, for example, to statements of the United States patent No.2,868,691 and No.3,095,355 in detail.

The injections for parenteral administration in this invention contain aseptic, aqueous or non-aqueous solutions, suspensions, or opalizers. For example, distilled water for injection and physiological saline are contained in aqueous solutions or suspensions. As non-aqueous solutions or suspensions, for example, the alcohol kind such as ethanols, propylene glycol, polyethylene glycols, vegetable oils such as olive oil, or polysorbate 80(registered trademark), etc., are enumerated. In addition, such compositions may contain supplements such as preservatives, moistening agents, emulsifiers, dispersants, stabilizers, or solubilizers. These are made aseptic by filtration with passing to bacteria removal filter, mixing and irradiation of sterilizers. Moreover, these can be used by being manufactured as sterilized solid compositions and then dissolved to distilled water for injection, which are made to sterility or sterility before use, or other solvents.

In other compositions for the parenteral administration, one or more activators and liquids for external use prescribed by usual methods, ointment drugs, coating drugs, suppositories for administering in intestinum rectum, or pessaries for administering in vagina, etc., are contained.

### BRIEF DESCRIPTION OF THE DRAWINGS

Figure 1 shows the amount of ACTH production in LPS-induced wild type (BL6) mice and EP₁ receptor-deficient(EP₁ -/-) mice.
Figure 2 shows the amount of ACTH production in LPS-induced wild type (BL6) mice on administering EP₁ antagonist.
Figure 3 shows decrease of spontaneous locomotor activity in LPS-induced wild type (BL6) mice and EP₁ receptor-deficient EP₁ -/-) mice.
Figure 4 shows the influence of EP₁ antagonist on decrease of spontaneous locomotor activity of LPS-induced wild type (WT) mice.
Figure 5 shows actions of jumping off platform of WT mice on administering EP₁ antagonist., wild mice(WT), and EP₁ or EP₁ receptor-defected mice.
Figure 6 shows the influence of EP₁ receptor-deficiency on the acoustic startle reactions of EP₁ receptor-deficient mice(●) and wild type mice(○mark).
Figure 7 shows the influence of EP₁ receptor-deficiency on aggressive behaviors of electroshock-induced EP₁ receptor-deficient mice(black) and wild type(WT) mice(grey).
Figure 8 shows the influence of EP₁ receptor-deficiency on social behaviors of EP₁ receptor-deficient mice and wild type(WT) mice.
Figure 9 shows the ratio(dopamin or serotonin is assumed to be 100.) of the metabolites to dopamin (DA) and serotonin (5-HT) of cerebral cortex in wild (WT)mice (white) and EP₁ receptor-deficient mice(black).
Figure 10 shows the ratio(dopamin or serotonin is assumed to be 100.) of the metabolites to dopamin (DA) and serotonin (5-HT) of striate corpus in wild (WT)mice (white) and EP₁ receptor-deficient mice(black).

### THE BEST MODE FOR CARRYING OUT THE INVENTION

For example, the following experiments were indicated that the administering EP₁ antagonists gave similar symptoms to PTSD.

### Experiment example 1: effects of EP₁ antagonists on ACTH production by LPS induction.

### [Experimental method]

0.1 mg/kg of LPS(lipopolysaccharide) as an infectious stress was intraperitoneally administered to C57BL/6 strain mice. The blood be gathered at 60 minutes later after administering LPS, the induced stress reaction, that is, activation of hypothalamus-pituitary-adrenal system were measured as a marker on ACTH in plasma. ACTH was measured with ACTH IRMA kit (Mitsubishi Yuka Medical Co., Tokyo, Japan). (4-[2-(N-isobutyl-2-furanylsulfonylamino)-5-trifluoromethylphenoxymethyl]cinna mic acid(the compound which had been described to example 18(94) in WO98/27053, was used.), which is EP1 antagonist, was administered 1 hour ago before administering LPS.

### [Experimental result]

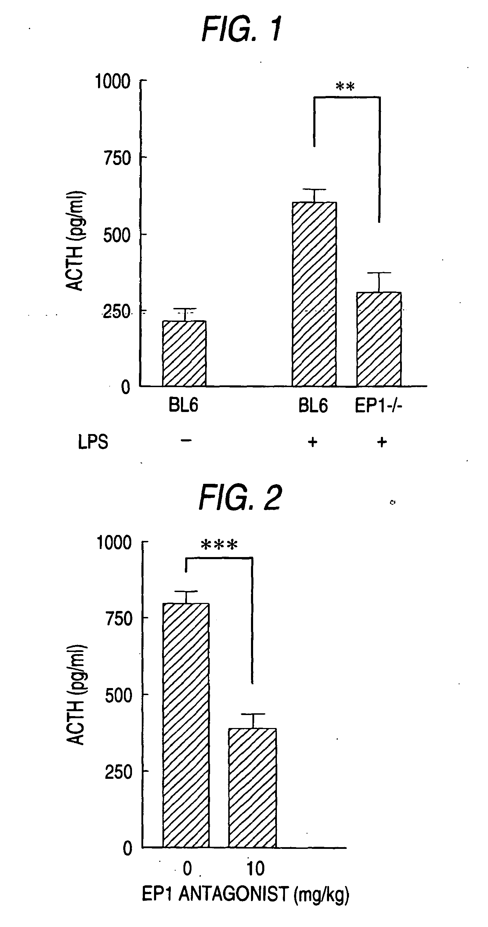

Table 1, figure 1, and figure 2 show the experimental results. The vertical axis in their figures present the amount of ACTH production. From figure 1, it is obvious that ACTH production are reinforced when LPS is administered to wild type (BL6) mice(BL6+), and the production significantly decreases in EP₁-deficient (EP₁-/-)mice, and from figure 2, it is obvious that the amount of ACTH production by LPS-induction significantly decreases when EP₁ antagonist is administered to wild type mice.

That is, ACTH production induced by administering LPS significantly decreased in the EP₁ antagonist administering group. This level is equal to that in EP1 receptor-deficient mice. It was suggested that the stress response by infectious stimulation could happen through EP₁ receptor. It is known that LPS causes diseased actions of heating, decrease of motor activity, loss appetite, and the increase of sleeping hours, etc., besides the above ACTH discharge. Then, the influence of EP₁ receptor on the decrease of momentum induced by administering LPS was examined (following experiment example 2).

**Table 1**

| | Concentration of ACTH in plasma (pg/ml) | Number of examples |
|---|---|---|
| Control (-LPS) | 211.6 ± 44.9 | 6 |
| BL/6 | 597.3 ± 45.2 | 6 |
| EP1-K0 | 300.3 ± 72.0 | 6 |
| EP2-K0 | 671.3 ± 52.4 | 6 |
| EP3-K0 | 337.8 ± 59.6 | 6 |
| EP4-K0 | 698.8 ± 181.9 | 5 |

### Experiment example 2: Influence of EP₁ antagonist on decrease of spontaneous locomotor activity by LPS induction.

### [Experimental method]

0.1mg/kg of LPS is intraperitoneally administered to C57BL/6 mice, they are put in acrylic cage(30×45×35cm(height), and the momentum for 12 hours from the 8 p.m. to 8 a.m. of next morning was measured with mouse action meter, Infrared photo beam counter(Neuroscience Co., Ltd., Tokyo, Japan). 0.1%(W/W) of EP₁ antagonist (the same as experiment example 1 was used) mixed with food was given. Similarly, the difference of the momentum by administering LPS were compared by using wild mice (WT) and EP₁ receptor-deficient mice.

### [Experimental result]

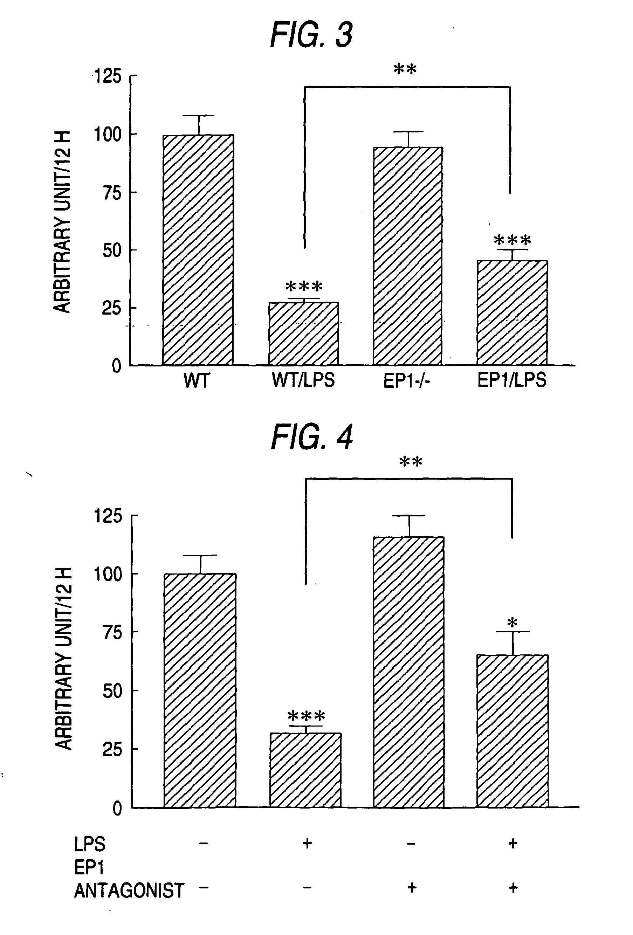

Figure 3 and figure 4 show the experiment result. The vertical axis in figures present the relative evaluation value of spontaneous locomotor activity in which LPS non-administrated group of wild type (WT) mice is assumed to be standard(100). They present that the ratio of the decrease of the spontaneous locomotor activity is few in EP₁-deficient mice, though the spontaneous locomotor activity decreases when LPS is administered to wild type mice.

When LPS was administered to EP₁ receptor-deficient mice, the decrease of the spontaneous locomotor activity was not so admitted compared to wild mice (VVT) (Figure 3). As well, the decrease of the spontaneous locomotor activity induced by LPS has decreased even when EP₁ antagonist is treated to a wild mice(Figure 4). Therefor, it was suggested that EP₁ receptor could take part in the behavioristic response by the infectious stress such as LPS, etc.

### Experiment example 3: the action of jumping off platform by EP₁ antagonist.

### [Experimental method]

Wild mice, EP₁ or EP₃ receptor-deficient mice, or WT mice to which 10 mg/kg of EP₁ antagonist(the same as experiment example 1 was used.) was intraperitoneally administered put on about 20cm high platform(beaker), and the presence of the action of jumping off was observed.

### [Experimental result]

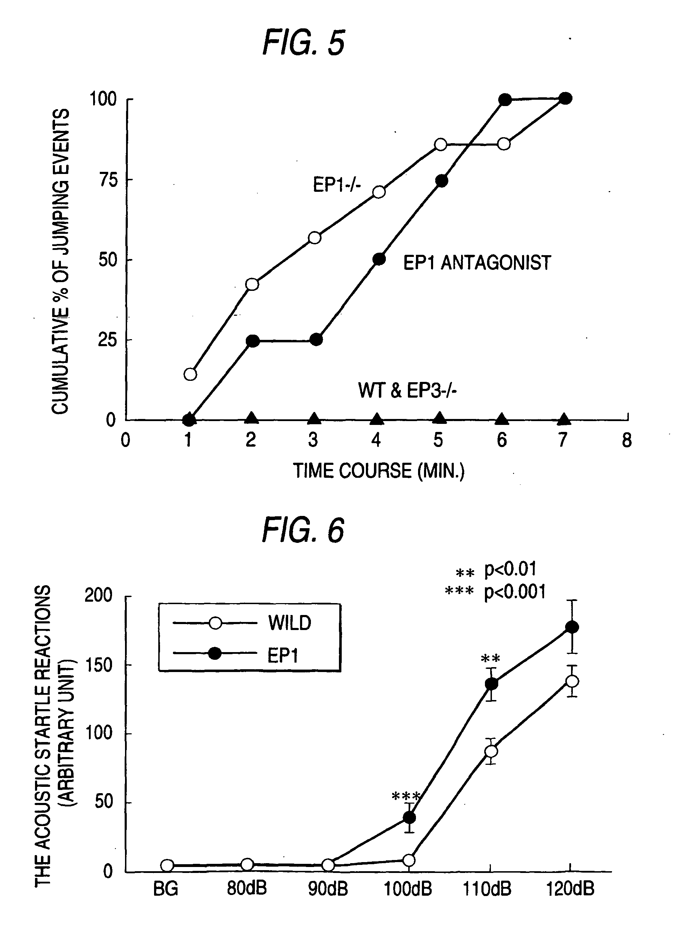

Figure 5 shows the experiment result. The horizontal axis in the figure presents the elapsed time, the vertical axis presents the ratio of the mice jumped down, and ▲ sign presents a data of wild type (Wild)mice (WT) and EP 3 receptor-deficient mice(EP₃-/-), ○ sign presents that of EP₁ receptor-deficient mice, and ● sign presents that of EP₁ antagonist administered wild type mice.

Wild mice stay on the plat even if they leave for 7 minutes, while some of EP₁ receptor-deficient mice jumped off from 1 minute later, and all jumped off at 7 minutes later. All mice in the EP₁ antagonist administrated group jumped off the platform 7 minutes later as well as EP₁ receptor-deficient mice.

### Experiment example 4: influence of EP₁ receptor-deficiency on the startle reaction to acoustic stimulation.

### [Experimental method]

The startle reaction to acoustic stimulation was induced by using SR-Lab system(San Diego Instruments, CA, USA) just as the method reported by K.Nakamura(Eur. J. Neurosci.13, 179-189, 2001). That is, wild mice(WT) or EP₁ receptor-deficient mice were put in a chamber, and tamed to sounds of 70dB for 10 minutes, acoustic stimulations of 80, 90, 10,110 or 120dB(6 times per 40 milliseconds) were given, then the presence of the startle reaction for 200 milliseconds was observed. The presence of the startle reaction was judged by measuring mice's muscle contraction through the electrode set up on the chamber floor.

### [Experimental result]

Figure 6 shows the experiment result. The horizontal axis in the figure presents the magnitude (dB) of the acoustic stimulation, the vertical axis presents the reaction (startle response) to the acoustic stimulation, and ● and ○ sign present the data of EP₁ receptor-deficient mice and wild type mice(Wild), respectively. Though the reaction to the acoustic stimulation in EP₁ receptor-deficient mice reinforced more than that in wild mice, it is known that the reinforcement of the reaction to this acoustic stimulation is admitted when the metabolism of PTSD and encephalon internal dopamine reinforces.

### Experiment example 5: Influence of EP₁ receptor-deficiency on aggressive behavior of electroshock-induced mice.

### [Experimental method]

After two male mice(wild mice(WT) or EP₁ receptor-deficient mice) were put in 3L of glass container and stimulated by the electroshock(1H, 200m seconds, 0.3mA for 3 minutes), these aggressive behaviors were recorded to the video. The time up to the first aggressive behavior(1 atency; second) and the attack frequency, and total attack time was compared.

### [Experimental result]

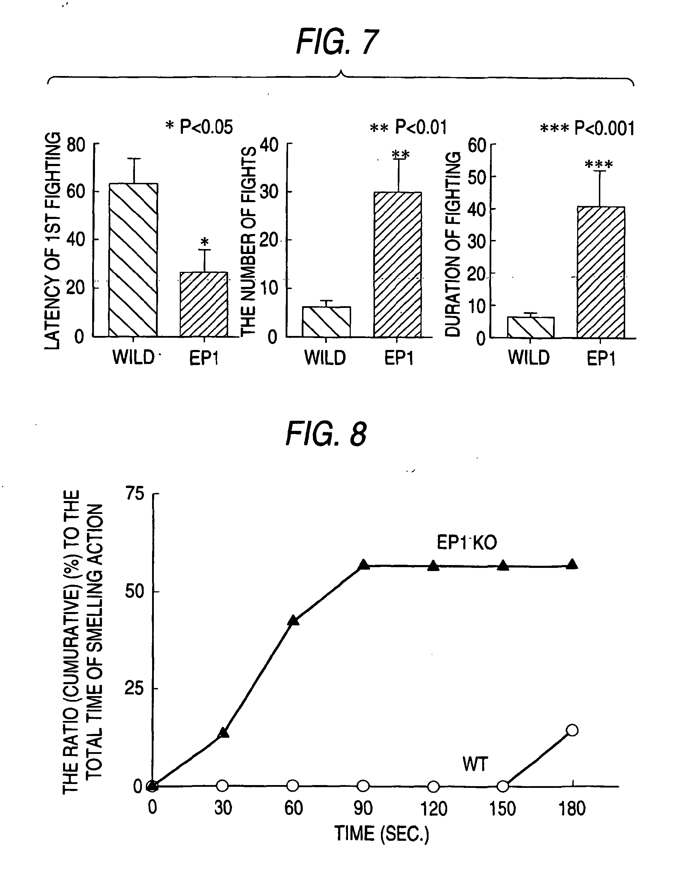

Figure 7 shows the experiment result. From the left in figure, (latency of 1st fighting) means the time up to the first attack, (the number of fights) means the attack frequency, and (duration of fighting) means total of attack time. The time up to the first attack of EP₁ receptor-deficient mice(black) was shorter, the attack frequency was more, and total attack time was also obviously longer than those of wild(WT) mice(grey).

### Experiment example 6: Influence EP₁ receptor-deficiency on mice's social behaviors

### [Experimental method]

The action admitted when wild mice(WT) or EP₁ receptor-deficient mice cohabit with young male mice (5 weeks of ages) was observed. Total times of the aggressive behavior (% of the observation time) and the smelling action (It is thought the social behavior that showed the concern) were measured, and compared.

### [Experimental result]

Figure 8 shows the experiment result. The vertical axis in figure presents the ratio (Cumurative) (%) to the total time of smelling action. When cohabiting with young males, wild (WT) mice (○ sign) show the smelling action and do not hardly show the aggressive behavior. It has been understood that this action could be a natural, social behavior, which they show the concern to a strange, young male. The time of the smelling action of EP₁ receptor-deficient mice were short and they frequently showed the aggressive behavior after some interval. Moreover, it was confirmed that the time of the smelling action was shortened by administering EP₁ antagonist to wild mice.

Therefor, it was suggested that by defect in EP₁ receptor, they lose the social behavior and suddenly become aggressive. And, it was confirmed that the aggressiveness could been further reinforced when LPS was administered to EP₁ receptor-deficient mice. That is, it was suggested that the abnormality of the social behavior, which was able to bring by failure of EP₁ receptor, could more actualize when they fall into weak pathema by administering LPS.

### Experiment example 7: influence of EP₁ receptor deficiency on in-cerebrum monoamine metabolism

### [Experimental method]

After treatment of microwave, brains removed from wild(WT) mice or EP1 receptor-deficient mice were drawn in various areas. The monoamine and the metabolite were extracted with 0.2M periodic acid, and measured in the HPLC.

### [Experimental result]

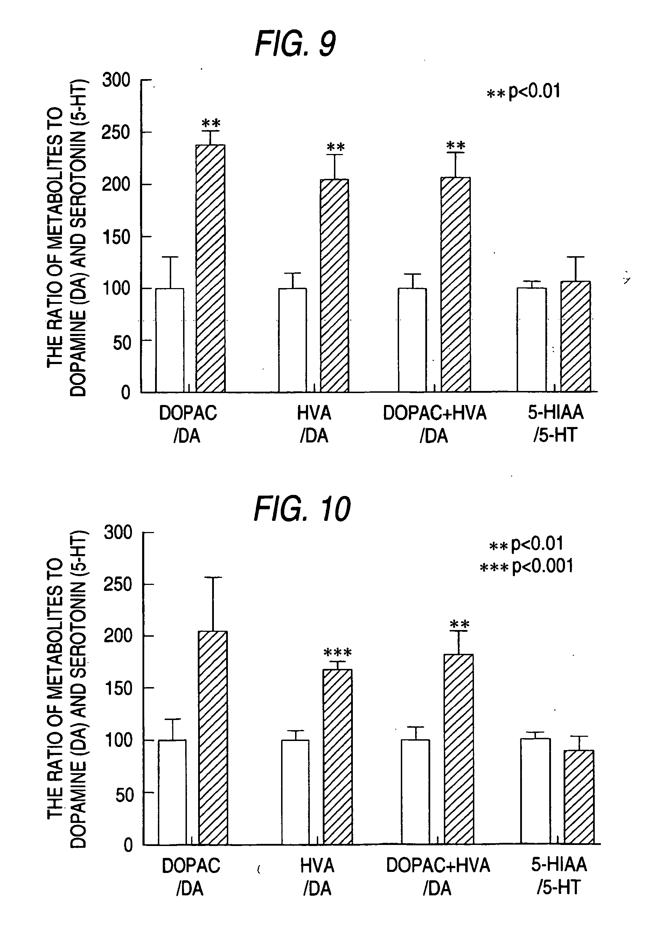

Figure 9 and figure 10 show the experiment result. The vertical axis in figure presents the ratio(dopamine or serotonin is assumed to be 100.) of metabolites to dopamine (DA) and serotonin (5-HT) of the cerebral cortex of EP₁ receptor-deficient mice. DOPAC presents 3,4-dihydroxyphenyl acetic acid, HVA presents 3-methoxy-4-hydroxy-phenyl acetate, both are metabolites of the DA(dopamine). 5-HIAA presents 5-hydroxyindolacetic acid, which is an metabolite of 5-HT (serotonin).

The serotonin metabolism is not so reinforced though the rate of the metabolite to dopamine in the cerebral cortex rises, and the dopamine metabolism reinforced in EP₁ receptor-deficient mice(Figure 9). The serotonin metabolism is not so reinforced though the rate of the metabolite to dopamine in the striate corpus rises, and the dopamine metabolism reinforced in EP₁ receptor-deficient mice(Figure 10). It is thought that these are related to an increase in aggressiveness and motor activity.

### Experiment example 8: Influence by haloperidol (dopamine antagonist) administering.

As a result of experiment similar to experiment example 6 by administering haloperidol to EP₁ receptor-deficient mice, it was confirmed that the time up to the first attack elongates, the attack frequency decreases, and the total of attack time shortens.

Furthermore, it was also confirmed that the spontaneous locomotor activity decreased when haloperidol was administered to EP₁ receptor-deficient mice.

Therefor, it is suggested that the aggressiveness decrease by antagonizing to dopamine.

### Experiment example 9: experiments concerning with c-Fos expression and localization of EP₁ in intracerebral neuron

### [Experimental method]

To immunologically detect the expression of c-Fos(c-FosIR), mice to which LPS was administered before 2 hours were intravenously perfused with physiologic saline kept in ice and treated with Zamboni solution (0.21%, 2, 4, 6-trinitrophenol and 2% paraformaldehyde in 0.1M sodium phosphate solution (pH7.3)) after anesthetized with pentobarbital.

Mice brain and hypophysis are removed and fixed to 4% paraformaldehyde (0.1M sodium phosphate solution (pH7.3, phosphate buffer)) for 10 hours, and incubated in cold 25% sucrose solution. 30µm slices were prepared and used as samples. Then, these slices were pre-treated with 1.5% normal goat serum in 0.1M sodium phosphate solution(pH7.3) containing 0.9% sodium chloride and 0.3% Triton X-100 (PBS-Triton) for 2 hours and first incubated in anti-c-Fos rabbit polyclonal antibody(2000 folds dilution; Ab-5, Oncogenomescience). They were continuously incubated with biotinated goat antibodies to rabbit IgG(200 folds dilution; Vector laboratory) for 2 hours and with avidin-biotin complexes(100 folds dilution)(Vectorstain ABC-PO kit; Vector laboratory) for 1 hour lastly. After washed by phosphate buffer, these samples were treated with 50mM tris hydrochloric acid(pH7.6) including 0.02% diaminobenzidine four hydrochlorides and 0.01% hydrogen peroxides. For the quantitative analysis, the number of c-Fos-IR-stained cells in the biggest nucleus (CeA,PVN,NTS) or adenohypopophysis was counted. The mean value in these two parts was assumed to the representative scores of each mouse. To immunostain (C) EP₁ and tyrosine hydroxylase(TH), mice anesthetized well were perfused with cold PBS containing protease inhibitors 10 µ of Mp-amidinophenyl-methanesulphonylfluoride and 1 µg/ml of leupeptin) for 5 minutes.

Following freezing brain in liquid nitorogen, the 10 µm slices were prepared. After the samples were first treated with 95% ethanol for 30 minutes at -20°C, they were treated with 100% acetone for 3 minutes at the room temperature and incubated with anti-TH mouse monoclonal antibodies diluted with PBS containing anti-EP₁ rabbit polyclonal antibodies (400 folds dilution) and 1%BSA (100 folds dilution), and lastly incubated with anti-rabbit or mouse secondary antibodies respectively labeled in Fluorescein (fluorescent molecule) and Texas red (Amasham, Inc.).

To check the specificity of anti-EP₁ antibodies, COS-7 cells, which are expressed with either of EP₁, EP₃, or LacZ protein, was fixed by the method similar to that of the cerebral slice samples. Then, they were immunostained by using anti-EP₁ antibodies. The fluorescence imagings were obtained with confocal laser operation microscope (BioRadMRC-1024).

### [Experimental result]

(A) As a result of immunofluorescent stain in native COS cells(Mock), or EP₁ or EP3 receptor-expressed COS cells with anti-EP₁ antibodies, EP₁ receptor expressed in cultured cells was specifically staind with anti-EP₁ antibodies, and neither stained in Mock nor EP₃ expressed cells.
(B) As a result of immunofluorescent stain with anti-EP₁ antibodies by using brain prepared from WT or EP₁ receptor-deficinet mice, specific immune reactions with anti-EP₁ antibodies were observed in striate corpus(CPu), paraventricular nucleus(PVN), and amygdaloid nucleus(CeA) neurons of WT mice. On the other hand, because the reaction was hardly seen in EP₁ receptor-deficient mice, it was suggested that the detected immune reaction could present EP₁ receptor.
(C) As a result of a double immunofluorescence staining with anti-EP₁ antibodies and TH in substantia nigra(substantia nigra;SN) of WT mice, EP₁ signals were seen on about 50% of TH containing neuron surface. Because EP₁ coexists with TH in a part of neuron, it was thought whether EP₁ signaling could influence on TH expression in TH containing neuron.
(D) Brain homogenates were made by WT mice and EP₁ receptor-deficient mice, and analyzed by western blot using anti-TH antibodies. TH immunoreactivity in EP₁ receptor deficient-mice rose to about 2-fold compared to that in WT mice.

### Example 1 of pharmaceutical preparation

After the following each element had been mixed by the law of the art, and compressed, and 100 tablets containing 0.5mg of an active ingredient were obtained.
· (13E)-(11α, 15S, 17S)-2, 5-ethano-6, 9-dioxo-11, 15-dihydro-17, 20-dimethylprosta-13-enoic acid · α-cyclodextrin 250mg(content 50mg)
· Carboxymethylcellulose calcium 200mg
· Magnesium stearate 100mg
· Crystallite cellulose 9.2g

### Example 2 of pharmaceutical preparation

After the following each element had been mixed by the law of the art, the solution was sterilized by the law of the art and filled every 1ml to a vial, and freeze-dried b by the law of the art, then 100 vials containing 0.2mg of active ingredient in a vial were obtained.
· (13E)-(11α, 15S, 17S)-2, 5-ethano-6, 9-dioxo-11, 15-dihydro-17, 20-dimethylprosta-13-enoic acid · α-cyclodextrin 100mg(content 20mg)
· Mannitol 5g
· Distilled water 100ml

## Claims

1. Use of a EP₁ agonist in the manufacture of a medicament for the treatment of post-traumatic stress disorder (PTSD).

2. Use according to claim 1, wherein the EP₁ agonist is (13E)-(11α, 15S, 17S)-2,5-ethano-6,9-dioxo-11,15-dihydro-17,20-dimethylprosta-13-enoic acid, PGE₁ or PGE₂.

## Patentansprüche

1. Verwendung eines EP₁-Agonisten bei der Herstellung eines Medikaments zur Behandlung einer akuten Belastungsreaktion (PTSD).

2. Verwendung nach Anspruch 1, wobei der EP₁-Agonist (13E)-(11α,15S,17S)-2,5-Ethano-6,9-dioxo-11,15-dihydro-17,20-dimethylprosta-13-ensäure, PGE₁ oder PGE₂ ist.

## Revendications

1. Utilisation d'un agoniste de EP₁ dans la fabrication d'un médicament pour le traitement d'un état de stress post-traumatique (ESPT).

2. Utilisation selon la revendication 1 où l'agoniste de EP₁ est l'acide (13E)-(11α, 15S, 17S)-2,5-éthano-6,9-dioxo-11,15-dihydro-17,20-diméthylprosta-13-énoïque, PGE₁ ou PGE₂.
